# EUROPEAN PATENT SPECIFICATION

(11) **EP 1 082 949 B1**
(45) Date of publication and mention of the grant of the patent: **20.07.2005**
(21) Application number: 00119513.0
(22) Date of filing: 07.09.2000
(51) Int. Cl.: A61F 2/34

(54) **Acetabulum prosthesis for the hip**
Gelenkpfannenprothese für die Hüfte
Prothèse acetabulaire pour la hanche

(30) Priority: 09.09.1999 IT UD990166
(43) Date of publication of application: 14.03.2001
(73) Proprietor: LIMA Lto SpA, 33030 Villanova di S. Daniele (UD) (IT)
(72) Inventor: DALLA PRIA, Paolo, 33100 UDINE (IT)
(74) Representative: Petraz, Gilberto Luigi

(56) References cited:
- EP-A- 0 091 315
- EP-A- 0 123 514
- EP-A- 0 532 439
- EP-A- 0 591 594
- EP-A- 0 807 426
- DE-U- 29 804 880
- FR-A- 2 719 465
- US-A- 5 658 338

## Description

### FIELD OF THE INVENTION

This invention refers to an acetabulum prosthesis suitable to be used in the field of prostheses to restore articulation to the hip in degenerative or traumatic pathologies.

### BACKGROUND OF THE INVENTION

The state of the art includes acetabulum prostheses by means of which articulation is restored to a hip which has been damaged following a degenerative or traumatic pathology. These prostheses usually comprise a cotyloid component, called an acetabulum cup, suitable to be made solid with the acetabulum seating of the hip, and an acetabulum insert suitable to be inserted into said acetabulum cup and to accommodate the spherical head of a femoral component which is attached to the femur.

The acetabulum insert is used to improve the tribological and mechanical characteristics of the prosthesis; for this purpose, it is made of specific materials with characteristics of minimum friction and high resistance to wear such as, for example, polyethylene.

So that the femur can move substantially as in a natural articulation, the acetabulum cup must be perfectly positioned and attached to the bone tissue defining the acetabulum seating of the hip.

This attachment is usually achieved by means of screws or nails, or also by simple mechanical interference when the natural seating allows.

To be more exact, the acetabulum cup must be attached inside the relative seating of the hip in such a manner on the one hand as to allow the femur to move in a natural way, and on the other hand so as to ensure an adequate covering for the head of the femoral component in order to prevent it from accidentally slipping out, such as may occur for example in the event of a dislocation.

As persons of skill in the art well know, the positioning of the acetabulum cup with respect to the relative seating of the hip is defined by two angles, called angle of abduction and angle of anteversion, which define the orientation in space of the acetabulum cup, normally consisting of a semi-spherical cap, with respect to planes of reference.

The state of the art includes the use of suitable devices to control the positioning which, during the operation, allow to verify the values of these angles before the acetabulum prosthesis is fitted definitively to the seating of the hip.

Often, however, the bone structure of the patient, due to degeneration, pathology or trauma he/she has suffered, does not allow to position the acetabulum cup correctly, which limits the mobility of the femoral articulation and does not ensure that the head of the femoral component is sufficiently covered, in the sense that it has an adequate surrounding surface.

A mistaken positioning of the acetabulum cup may also entail the problem of an anomalous contact between the head of the femoral component and the relative seating of the acetabulum insert, with consequent problems of excessive pressure, which may cause wear on the surface of the seating and/or may cause the head to accidentally slip out of the relative seating, with serious problems for the patient.

FR-A-2.719.465 discloses an acetabulum prosthesis consisting of three elements, respectively an outer cap which is accommodated and fixed in the patient's acetabulum cavity, an intermediate module attached to said outer cap and an inner cap inside which the head of the femoral component is inserted and can rotate.

This document however does not provide that the intermediate module can be oriented and clamped in different spatial positions with respect to the outer cap, according to various requirements on different occasions; on the contrary, it provides that the intermediate module can be clamped through interference in a single position with respect to the outer cap, and this does not solve the problems outlined above.

Document EP-A-123.516 describes an acetabulum prosthesis comprising an outer cap which is accommodated and fixed in the patient's acetabulum cavity, an intermediate module consisting of two components, a first component to couple with said outer cap and a second component able to receive the head of the femoral component, and an element of reciprocal attachment, of the auxiliary flange type, which allows to direct and clamp the intermediate module through interference in different spatial positions with respect to the outer cap.

Such a clamping system with an auxiliary flange has a plurality of disadvantages.

First of all, there is a greater risk of mechanical failure, since the flange is subjected to dynamic stress which can cause breakage through fatigue. Moreover, since the intermediate module is free to rotate, it might move, during the step when the flange is attached, from the position set by the surgeon.

The clamping of the intermediate module can also be made unstable if only one of the circumferential screws on the flange comes loose, with potentially very dangerous effects on the entire prosthesis.

To ensure stable clamping, the locking torque of the flange screws must be very high, since the holding power of the prosthesis is closely linked to the friction which is produced between the flange and the intermediate module, due to the torsional force which is transmitted by the femoral head to the interface between the flange and the intermediate module.

Due to its design structure, the intermediate module must protrude towards the stem of the prosthesis, so that the flange can have a spherical surface adequate to hold the intermediate module through interference with the outer cap. This causes a high risk of accidental contact between the stem of the prosthesis and the intermediate module, with a serious risk of dislocation of the femoral head and an abrasion of the metal surfaces, which leads to a possible release of debris and to risks of breakage through fatigue.

The design structure, with the intermediate module having a spherical extension which exceeds the equator so that it can be clamped by the flange, also imposes a reduction in the usable space with regard to the minimum thickness of the articular insert (component which accommodates the femoral head), with considerable risks of premature wear or breakage through fatigue of the plastic material of which it is made.

The present Applicant has devised and embodied this invention to overcome these problems and to obtain further advantages.

### SUMMARY OF THE INVENTION

The invention is set forth and characterized in the main claim, while the dependent claims describe other characteristics of the invention.

The purpose of the invention is to achieve an acetabulum prosthesis which will allow, substantially irrespective of the bone structure of the patient and the type of degeneration or trauma suffered, a correct positioning of the acetabulum component so as to allow the femur to move substantially as in a natural articulation.

A further purpose of the invention is to allow the acetabulum cup to be positioned so as to ensure the head of the femoral component is properly covered, in order to reduce risks of it accidentally slipping out.

In accordance with these purposes, an acetabulum prosthesis according to the invention consists of at least three components:
- an outer shell suitable to be inserted and made solid with the acetabulum seating of the hip,
- an intermediate element suitable to be inserted inside said outer shell and
- an acetabulum insert suitable to be inserted inside the intermediate element and to accommodate the spherical head of the femoral component.

The outer shell has an outer conformation suitable to be coupled with the natural acetabulum seating of the hip and an inner conformation suitable to house the intermediate element.

The intermediate element has an inner conformation suitable to house the acetabulum insert, which in turn is equipped with a cavity suitable to accommodate the head of the femoral component.

According to the invention, the intermediate element is suitable to be made to rotate and/or oscillate in the insertion seating of the outer shell, after the latter has been attached to the natural seating of the hip, in order to identify a correct position thereof; this position is chosen from among a plurality of selectable discreet positions, and respects the necessary requirements: that is, articulated mobility of the femur and sufficient covering of the head of the femoral component.

Once the correct spatial position with respect to the outer shell has been identified, the intermediate element is able to be attached stably to the outer shell, by means of a direct coupling and without requiring additional, auxiliary outer elements, such as flanges or similar.

The outer shell can therefore be attached in the position allowed by the pathological conditions of the natural seating of the hip, so as to ensure a secure and stable anchorage in all cases.

Any possible incorrect positioning of the outer shell can then be compensated by rotating or oscillating the intermediate element with respect thereto until a position is found which respects the necessary requirements; when this position has been found, the intermediate element is stably and directly attached to the outer shell.

To this end, the acetabulum prosthesis is equipped with constraining means suitable to stably clamp the intermediate element with respect to the outer shell, thus defining the insertion position of the relative acetabulum insert.

The constraining means allow to adjust the position of the intermediate element with respect to the outer shell with respect to at least one reference plane, advantageously with respect to at least two planes substantially orthogonal to each other.

According to another characteristic of the invention, the constraining means allow to position and clamp the intermediate element both before and after the outer shell has been attached to the relative seating of the hip.

In a preferential embodiment, the constraining means comprise a plurality of through apertures distributed on the surface of the outer shell, a plurality of through holes distributed on the intermediate element and clamping elements suitable to be inserted and clamped in specific pairs of through apertures and holes aligned with each other after the correct position of the intermediate element has been found.

In one embodiment of the invention, the clamping elements consist of screws and the apertures and at least the holes made on the outer shell are threaded. According to a variant, the clamping elements consist of rivets or trigger devices.

In this last embodiment, the through apertures of the outer shell may consist of eyelets which allow a greater number of working positions of the intermediate element.

In the preferential embodiment of the invention, the position of the intermediate element with respect to the outer shell may be adjusted so as to cover an angle of abduction of between 0° and 40° and an angle of anteversion also between 0° and 40° with respect to the reference planes.

The acetabulum prosthesis according to the invention thus always allows to obtain a correct positioning of the acetabulum insert irrespective of the traumatic or degenerative condition of the natural seating of the hip inside which the outer shell is attached.

### BRIEF DESCRIPTION OF THE DRAWINGS

These and other characteristics of the invention will become clear from the following description of some preferred forms of embodiment given as a non-restrictive example with reference to the attached drawings wherein:
Fig. 1 is an exploded section view of an acetabulum prosthesis according to the invention;
Fig. 2 shows a section of the prosthesis in Fig. 1 in its assembled condition;
Fig. 3 is a three dimensional view of the prosthesis as in Fig. 1;
Fig. 4 is a side view of the prosthesis according to the invention;
Fig. 5 is a view from below of the prosthesis according to the invention without the acetabulum insert;
Fig. 6 is a view from above of the prosthesis according to the invention;
Fig. 7 shows a variant of Fig. 6.

### DETAILED DESCRIPTION OF SOME PREFERRED FORMS OF EMBODIMENT

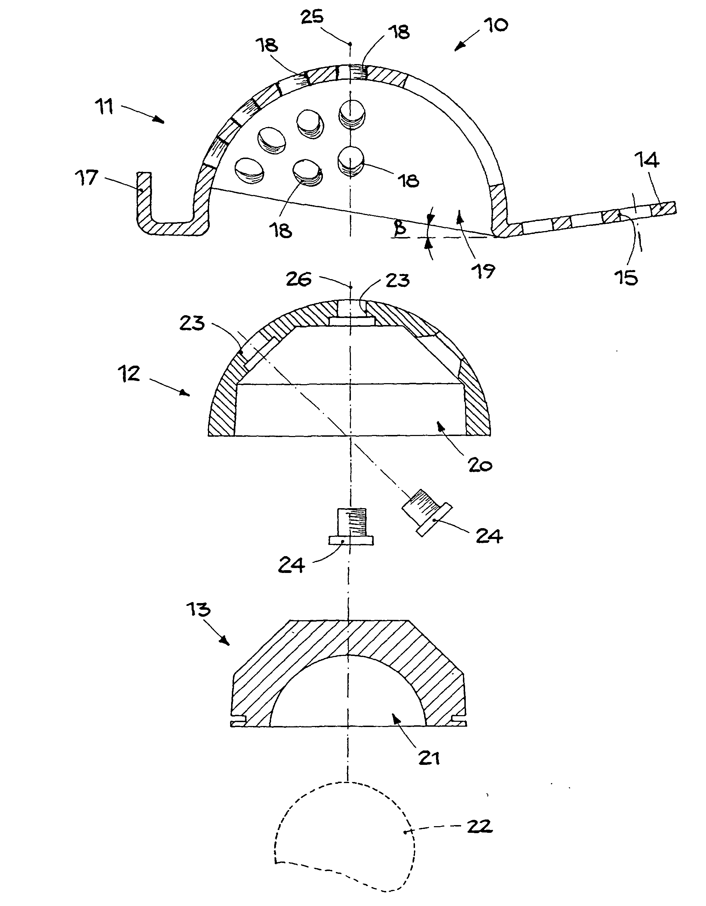

Fig. 1 is an exploded view of an acetabulum prosthesis 10 according to the invention which comprises an outer shell 11, an intermediate element 12 and an acetabulum insert 13.

The outer shell 11 may be made of any appropriate material, for example titanium, and has a hemispherical outer conformation suitable to be inserted and anchored in a natural acetabulum seating of the hip.

To this end, the outer shell 11 is equipped with coupling means to couple with the bone tissue of the hip, in this case consisting of a hook 17 and three fins 14 equipped with holes 15, which allow suitable screws or nails to be inserted into the bone tissue of the hip seating.

The outer shell 11 is equipped with a plurality of threaded through holes 18 appropriately distributed over a substantial part of the hemispherical surface, and defines inside a seating 19 suitable to accommodate the intermediate element 12.

The intermediate element 12, which can be made of any appropriate material, for example titanium, has a hemispheric conformation mating with the seating 19 and defines inside a seating 20 suitable to accommodate the acetabulum insert 13.

The insert 13 has a conventional conformation and is equipped with a hemispheric cavity 21; the spherical head 22, shown by a line of dashes in Fig. 1, of a femoral component which is not shown here, is suitable to be inserted therein.

The intermediate element 12 is equipped with a plurality of through holes 23 suitably distributed over a substantial part of the hemispheric surface, inside which holes 23 screws 24 are suitable to be inserted, in this case of the type with a recessed hexagon head.

In the procedure to apply the acetabulum prosthesis according to the invention, the outer shell 11 is inserted into the acetabulum seating of the hip and anchored therein in a stable manner by means of the hook 17, the fins 14 and optional screws or nails.

The spatial orientation of the outer shell 11 depends on the conditions of the natural seating which, in a pathological condition of degeneration or trauma, may be partly damaged, deformed or incomplete, and therefore may not allow the shell 11 to be correctly positioned.

Once the shell 11 has been attached, the intermediate element 12 is inserted into the inner seating 19, and is free to rotate on several planes inside said seating 19. This allows the element 12 to be located in a correct working position, which to a certain extent is independent of the position taken by the outer shell 11 in the acetabulum seating of the hip.

Once this working position has been identified so as to respect the necessary requirements (that is, articulated femoral mobility and sufficient covering of the head of the femoral component), it is fixed by constraining means which make the intermediate element 12 solid with the outer shell 11, achieving a direct coupling which does not require any auxiliary means, such as flanges or similar.

The acetabulum insert 13 is then inserted inside said intermediate element 12.

In this case, the constraining means consist of screws 24, through holes 23 in the intermediate element 12, and threaded holes 18 in the outer shell 11.

The screws 24 are inserted and tightened respectively into the holes 23 and the holes 18 which are aligned after the correct working position has been found and adjusted.

This type of coupling defines a plurality of discreet positions of spatial orientation of the intermediate element 12, which the surgeon can choose on different occasions according to the type and conditions of application.

In this way, once the intermediate element 12 has been placed in the chosen position, and the clamping step has been started, the intermediate element 12 cannot move from said position any more, thus facilitating the clamping procedure and stability.

Moreover, the intermediate element 12 is not stressed by any torsional component, therefore a moderate locking torque of the screws 24 is sufficient to ensure that the clamping is stable over time.

With such a configuration, moreover, the hemispheric surface of the intermediate element 12 does not have to extend beyond the equator, therefore the risks of knocking against the femoral stem are reduced and no constraints are created on the thickness of the material which makes up the acetabulum insert 13.

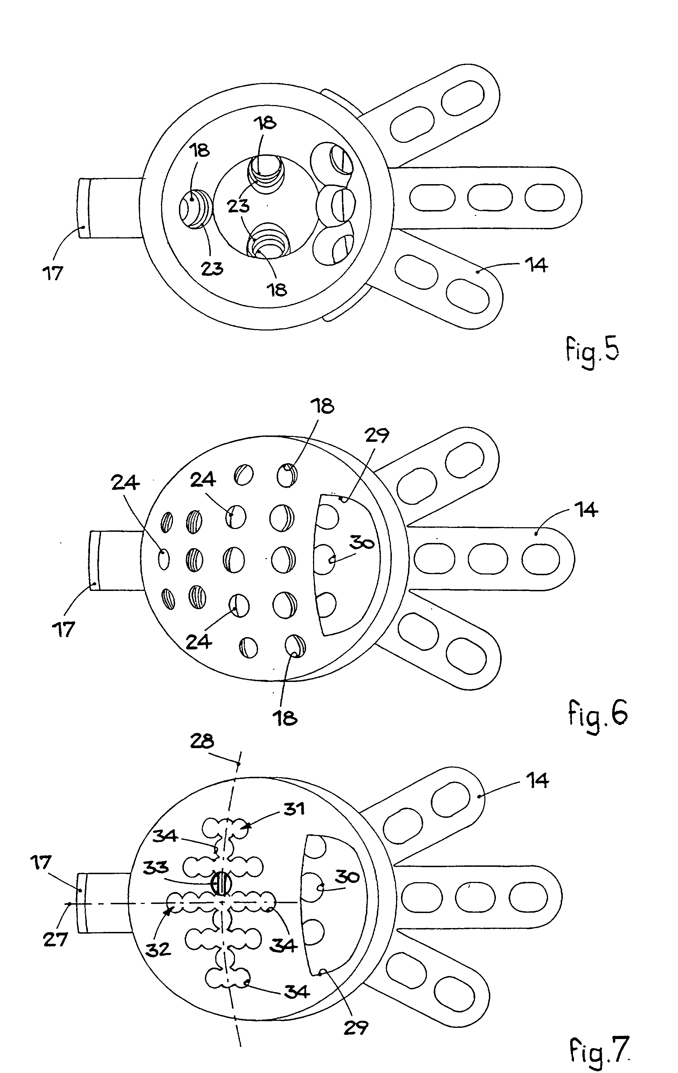

Fig. 5 shows a working position wherein the median axis 25 of the outer shell 11 and the median axis 26 of the intermediate element 12 coincide.

In this position the three through holes 23 of the intermediate element 12 are aligned with three threaded holes 18 of the outer shell 11, allowing to use three screws 24 which are not shown in Fig. 5, for reasons of simplicity.

According to the invention, the intermediate element 12 with the relative acetabulum insert 13 may take up a plurality of other working positions, rotated in space with respect to the outer shell 11, since the relative holes 18 and 23 are disposed for reciprocal clamping in all said plurality of positions.

To be more exact, the intermediate element 12 is free to rotate both on a plane 27 substantially frontal to the hip, in order to vary the angle of abduction, and also on a sagittal plane 28, substantially orthogonal to the frontal plane 27, and can then be clamped once the correct working position has been identified.

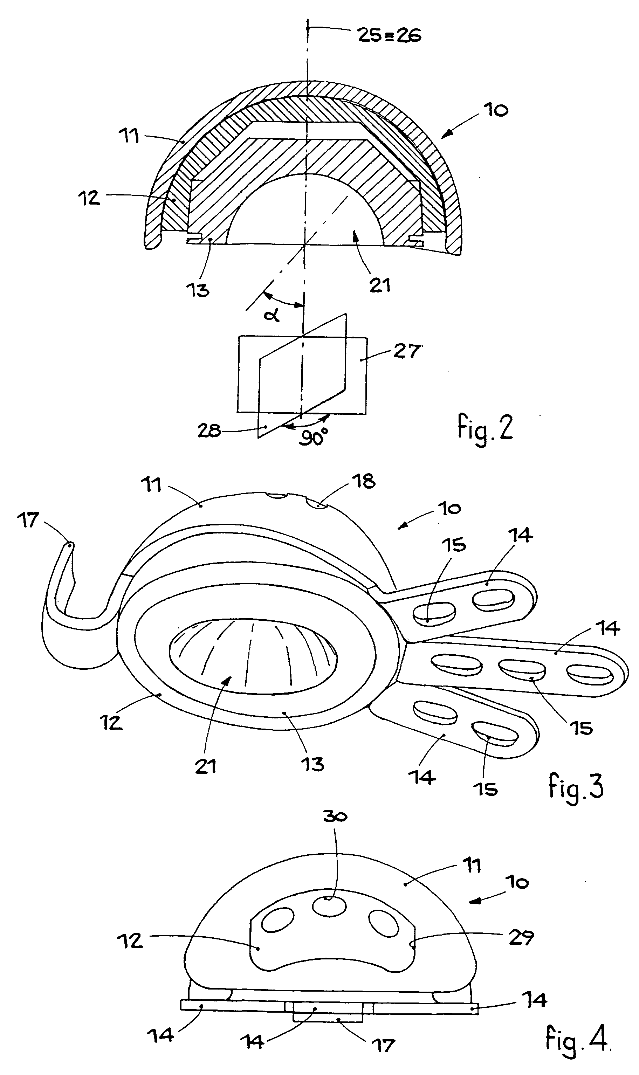

To this end, the holes 18 and 23 are distributed, as shown in Fig. 2, in such a manner that the intermediate element 12 can rotate inside the seating 19 of the outer shell 11 with respect to said frontal plane 27 and/or said sagittal plane 28, to cover an angle of abduction α, referred to the median longitudinal axis 25 of the shell 12, between 0° and 40°, and an angle of anteversion which is also between 0° and 40°.

The angle of anteversion is not shown in the Figures since it is defined on the plane orthogonal to the plane on which the angle of abduction is defined.

In this case, the outer shell 11 is also equipped with a window 29 and the intermediate element 12 with flared through holes 30.

Supplementary bone screws, not shown here, can be inserted into the flared through holes 30, and are suitable to improve the attachment of the acetabulum prosthesis to the bone tissues of the hip.

These supplementary screws, passing through the window 29, can be screwed to the bone tissue of the hip even after the intermediate element 12 has been definitively positioned and clamped to the outer shell 11.

In the variant shown in Fig. 7, instead of the holes 18 in the outer shell 12, there are intersecting eyelets 31 and 32 with their axes arranged respectively on a frontal plane 27 and on a sagittal plane 28.

The eyelets 31 and 32 are defined by a plurality of holes 34 made one after the other with a pitch less than the value of their diameter.

In this case, instead of the holes 23, the intermediate element 12 is equipped with a trigger device 33 suitable to be inserted and clamped selectively in any hole 34 whatsoever, to define the working position of the intermediate element 12.

The outer shell 11 can also have the lower surface inclined by an angle β with respect to the horizontal, thus allowing to improve the positioning of the acetabulum component (Fig. 1).

It is obvious that modifications and additions may be made to the acetabulum prosthesis 10 as described heretofore, but these shall remain within the field and scope of the invention. For example, the outer conformation of the shell 11, instead of hemispherical, can be cylindrical, conical, truncated cone or otherwise.

The outer conformations of the intermediate element 12 and the acetabulum insert 13 may also be different from those shown, provided that they mate with the respective housing seatings 19 and 20.

Or instead of the screws 24 or the trigger device 33, it is possible to use other clamping elements such as, for example, rivets or similar.

It is also obvious that the acetabulum prosthesis 10 according to the invention can also be used as a conventional prosthesis by eliminating the intermediate element 12 and by cementing the acetabulum insert 13 directly into the seating 19 of the shell 11.

Or the acetabulum insert 13 can be of a different type from that shown, such as for example of the type equipped with means to retain the spherical head 22 of the femoral component.

It is also obvious that, although the description refers to a specific example, a skilled person in the art shall certainly be able to achieve many other equivalent forms of an acetabulum prosthesis within the scope of the invention as defined by the appended claims.

## Claims

1. Acetabulum prosthesis for the hip comprising at least an outer shell, suitable to be inserted and attached in a relative natural acetabulum seating of the hip and defining inside a seating (19), and an acetabulum insert (13) equipped with a seating (21) suitable to accommodate the upper end or head (22) of a femoral component, also comprising an intermediate element (12) suitable to be inserted into said seating (19) in an intermediate position between said outer shell (11) and said acetabulum insert (13), said intermediate element (12) defining inside a housing seating (20) for said acetabulum insert (13) and being suitable to rotate in said seating (19) on at least one plane with respect to said outer shell (11), the prosthesis being **characterized in that** constraining means are provided to position and clamp said intermediate element (12), by means of reciprocal direct coupling, in a desired position, rotated with respect to said outer shell (11) and chosen from a plurality of selectable discreet positions, to define a specific working position for said intermediate element (12) and for the acetabulum insert (13) associated therewith.

2. Acetabulum prosthesis as in Claim 1, **characterized in that** said constraining means comprise a plurality of through holes (23) provided on said intermediate element (12), a plurality of threaded holes (18) provided on said outer shell (11) and screws (24) suitable to be inserted and tightened respectively into the through holes (23) and the threaded holes (18) which are aligned when said intermediate element (12) is taken to a desired working position with respect to said outer shell (11).

3. Acetabulum prosthesis as in Claim 1, **characterized in that** said constraining means comprise through apertures provided on said intermediate element (12) and through apertures provided on said outer shell (11), rivets or similar attachment elements being suitable to be inserted and clamped in the through apertures of said intermediate element (12) and of said outer shell (11) which are aligned when said intermediate element (12) is taken to a desired working position with respect to said outer shell (11).

4. Acetabulum prosthesis as in Claim 1, **characterized in that** said constraining means comprise at least a trigger device (33) provided on said intermediate element (12) and through apertures provided on said outer shell (11), said trigger device (33) being suitable to be inserted and clamped in said through apertures.

5. Acetabulum prosthesis as in Claim 3 or 4, **characterized in that** said through apertures provided on said outer shell (11) consist of eyelets (31, 32).

6. Acetabulum prosthesis as in Claim 5, **characterized in that** it provides at least a first eyelet (31) with the longitudinal axis arranged on said sagittal plane (28) and at least a second eyelet (31) with the longitudinal axis arranged on said frontal plane (27).

7. Acetabulum prosthesis as in Claim 5, **characterized in that** each eyelet (31, 32) is defined by a plurality of through holes (34) made one after the other with a pitch less than their diameter so as to be inter-communicating.

8. Acetabulum prosthesis as in Claim 1, **characterized in that** said constraining means are suitable to allow said intermediate element (12) to rotate inside said seating (19) of said outer shell (11) on at least a frontal plane (27) and on at least a sagittal plane (28) substantially orthogonal to each other.

9. Acetabulum prosthesis as in Claim 8, **characterized in that** said constraining means are suitable to allow said intermediate element (12) to rotate inside said seating (19) of said outer shell (11) with respect to said frontal plane (27) and/or said sagittal plane (28) to cover an angle of abduction (α) of between 0° and 40° and an angle of anteversion of between 0° and 40°.

10. Acetabulum prosthesis as in Claim 1, **characterized in that** said constraining means are suitable to allow said intermediate element (12) to rotate and be clamped both before and after the attachment of said outer shell (11) to said acetabulum seating of the hip.

11. Acetabulum prosthesis as in Claim 1, **characterized in that** said outer shell (11) is equipped with at least a window (29) and said intermediate element (12) is equipped with supplementary holes (30) into which screws are suitable to be introduced suitable to be screwed to the bone tissue of said acetabulum seating of the hip passing through said window (29) even after said intermediate element (12) has been definitively positioned and clamped.

12. Acetabulum prosthesis as in Claim 1, **characterized in that** said outer shell (11) is equipped with at least a hook (17) and/or fins (14) to anchor said outer shell (11) to the acetabulum seating of the hip.

## Patentansprüche

1. Hüftgelenksprothese für die Hüfte, umfassend mindestens eine Außenschale, die dafür geeignet ist, um in einem jeweiligen natürlichen Hüftgelenkssitz der Hüfte eingesetzt und angebracht zu werden, und die im Innern einen Sitz (19) begrenzt, und einen Hüftgelenkseinsatz (13), der mit einem Sitz (21) ausgerüstet ist, der dafür geeignet ist, um das obere Ende oder Kopf (22) einer Öberschenkelknochenkomponente aufzunehmen, auch umfassend ein Zwischenelement (12), das dafür geeignet ist, um in dem Sitz (19) in einer Zwischenposition zwischen der Außenschale (11) und dem Hüftgelenkseinsatz (13) eingesetzt zu werden, wobei das Zwischenelement (12) im Innern einen Unterbringungssitz (20) für den Hüftgelenkseinsatz (13) begrenzt und dafür geeignet ist, um sich in dem Sitz (19) in mindestens einer Ebene in Bezug zu der Außenschale (11) drehen zu lassen, wobei die Prothese **dadurch gekennzeichnet ist, dass** Zwangseinrichtungen bereitgestellt werden, um das Zwischenelement (12) mittels einer wechselseitigen direkten Kopplung in einer gewünschten Position zu positionieren und festzuklemmen, die in Bezug zu der Außenschale (11) gedreht ist und aus einer Mehrzahl von auswählbaren diskreten Positionen ausgewählt ist, um eine spezifische Arbeitsposition für das Zwischenelement (12) und für den Hüftgelenkseinsatz (13), der damit verbunden ist, festzulegen.

2. Hüftgelenksprothese nach Anspruch 1, **dadurch gekennzeichnet, dass** die Zwangseinrichtungen umfassen: eine Mehrzahl von Durchgangslöchern (23), die auf dem Zwischenelement (12) vorgesehen sind, eine Mehrzahl von Gewindelöchern (18), die auf der Außenschale (11) vorgesehen sind, und Schrauben (24), die dafür geeignet sind, um in den Durchgangslöchern (23) und den Gewindelöchern (18) eingesetzt bzw. festgezogen zu werden, die ausgerichtet werden, wenn das Zwischenelement (12) zu einer gewünschten Arbeitsposition in Bezug zu der Außenschale (11) gebracht wird.

3. Hüftgelenksprothese nach Anspruch 1, **dadurch gekennzeichnet, dass** die Zwangseinrichtungen Durchgangsöffnungen, die auf dem Zwischenelement (12) vorgesehen sind, und Durchgangsöffnungen, die auf der Außenschale (11) vorgesehen sind, umfassen, wobei Niete oder ähnliche Anbringelemente dafür geeignet sind, um in den Durchgangsöffnungen des Zwischenelements (12) und der Außenschale (11) eingesetzt und festgeklemmt zu werden, die ausgerichtet werden, wenn das Zwischenelement (12) zu einer gewünschten Arbeitsposition in Bezug zu der Außenschale (11) gebracht wird.

4. Hüftgelenksprothese nach Anspruch 1, **dadurch gekennzeichnet, dass** die Zwangseinrichtungen umfassen: mindestens eine Auslösevorrichtung (33), die auf dem Zwischenelement (12) vorgesehen ist, und Durchgangsöffnungen, die auf der Außenschale (11) vorgesehen sind, wobei die Auslösevorrichtung (33) dafür geeignet ist, um in den Durchgangsöffnungen eingesetzt und festgeklemmt zu werden.

5. Hüftgelenksprothese nach Anspruch 3 oder 4, **dadurch gekennzeichnet, dass** die Durchgangsöffnungen, die auf der Außenschale (11) vorgesehen sind, aus Öhren (31, 32) bestehen.

6. Hüftgelenksprothese nach Anspruch 5, **dadurch gekennzeichnet, dass** sie bereitstellt: mindestens ein erstes Öhr (31), dessen Längsachse in der Sagittalebene (28) angeordnet ist, und mindestens ein zweites Öhr (31), dessen Längsachse in der Frontalebene (27) angeordnet ist.

7. Hüftgelenksprothese nach Anspruch 5, **dadurch gekennzeichnet, dass** jedes Öhr (31, 32) durch eine Mehrzahl von Durchgangslöchern (34) begrenzt wird, das eines nach dem anderen mit einem Teilungsabstand hergestellt ist, der kleiner als ihr Durchmesser ist, so dass sie miteinander in Verbindung stehen.

8. Hüftgelenksprothese nach Anspruch 1, **dadurch gekennzeichnet, dass** die Zwangseinrichtungen dafür geeignet sind, um zu ermöglichen, dass sich das Zwischenelement (12) im Innern des Sitzes (19) der Außenschale (11) in mindestens einer Frontalebene (27) und in mindestens einer Sagittalebene (28), die im Wesentlichen orthogonal zueinander sind, drehen lässt.

9. Hüftgelenksprothese nach Anspruch 8, **dadurch gekennzeichnet, dass** die Zwangseinrichtungen dafür geeignet sind, um zu ermöglichen, dass sich das Zwischenelement (12) im Innern des Sitzes (19) der Außenschale (11) in Bezug zu der Frontalebene (27) und/oder der Sagittalebene (28) drehen lässt, um einen Abduktionswinkel (α) von zwischen 0° und 40° und einen Anteversionswinkel von zwischen 0° und 40° abzudecken.

10. Hüftgelenksprothese nach Anspruch 1, **dadurch gekennzeichnet, dass** die Zwangseinrichtungen dafür geeignet sind, um zu ermöglichen, dass sich das Zwischenelement (12) sowohl vor als auch nach der Anbringung der Außenschale (11) an dem Hüftgelenkssitz der Hüfte drehen lässt und festgeklemmt wird.

11. Hüftgelenksprothese nach Anspruch 1, **dadurch gekennzeichnet, dass** die Außenschale (11) mit mindestens einem Fenster (29) ausgerüstet ist und das Zwischenelement (12) mit Zusatzlöchern (30) ausgerüstet ist, in die Schrauben zur Einführung passen, die geeignet sind, um an das durch das Fenster (29) hindurchtretende Knochengewebe des Hüftgelenkssitzes der Hüfte geschraubt zu werden, selbst nachdem das Zwischenelement (12) endgültig positioniert und festgeklemmt worden ist.

12. Hüftgelenksprothese nach Anspruch 1, **dadurch gekennzeichnet, dass** die Außenschale (11) mit mindestens einem Haken (17) und/oder Rippen (14) ausgerüstet ist, um die Außenschale (11) an dem Hüftgelenkssitz der Hüfte zu verankern.

## Revendications

1. Prothèse acétabulaire pour la hanche, comprenant au moins une coque externe pouvant être insérée et fixée dans un siège acétabulaire naturel correspondant de la hanche et définissant intérieurement un siège (19), et un insert acétabulaire (13) pourvu d'un siège (21) pouvant recevoir l'extrémité ou la tête supérieure (22) d'un composant fémoral, comprenant également un élément intermédiaire (12) pouvant être inséré dans ledit siège (19) en position intermédiaire entre ladite coque externe (11) et ledit insert acétabulaire (13), ledit élément intermédiaire (12) définissant à intérieurement un siège d'emboîtement (20) pour ledit insert acétabulaire (19) et pouvant pivoter dans ledit siège (13) sur un plan au moins par rapport à ladite coque externe (11), la prothèse **se caractérisant en ce que** des moyens de contrainte sont prévus pour positionner et serrer ledit élément intermédiaire (12), au moyen d'un accouplement direct réciproque, dans la position souhaitée, en rotation par rapport à ladite coque externe (11) et choisie parmi une pluralité de positions discrètes possibles, afin de définir une position de travail particulière pour ledit élément intermédiaire (12) et pour l'insert acétabulaire (13) qui lui est associé.

2. Prothèse acétabulaire selon la revendication 1, **caractérisée en ce que** lesdits moyens de contrainte comprennent une pluralité de trous traversants (23) ménagés sur ledit élément intermédiaire (12), une pluralité de trous filetés (12) ménagés sur ladite coque externe (11), et des vis (24) pouvant être insérées et serrées respectivement dans les trous traversants (23) et les trous filetés (18) qui sont alignés lorsque ledit élément intermédiaire (12) est amené en position de travail souhaitée par rapport à ladite coque externe (11).

3. Prothèse acétabulaire selon la revendication 1, **caractérisée en ce que** lesdits moyens de contrainte comprennent des orifices traversants ménagés sur ledit élément intermédiaire (12), et des orifices traversants ménagés sur ladite coque externe (11), des rivets ou des éléments de fixation similaires pouvant être insérés et serrés dans les orifices traversants dudit élément intermédiaire (12) et de ladite coque externe (11) qui sont alignés lorsque ledit élément intermédiaire (12) est amené en position de travail souhaitée par rapport à ladite coque externe (11).

4. Prothèse acétabulaire selon la revendication 1, **caractérisée en ce que** lesdits moyens de contrainte comprennent au moins un dispositif de déclenchement (33) prévu sur ledit élément intermédiaire (12) et des orifices traversants ménagés sur ladite coque externe (11), ledit dispositif de déclenchement (33) pouvant être inséré et serré dans lesdits orifices traversants.

5. Prothèse acétabulaire selon la revendication 3 ou 4, **caractérisée en ce que** lesdits orifices traversants ménagés sur ladite coque externe (11) sont constitués d'oeillets (31, 32).

6. Prothèse acétabulaire selon la revendication 5, **caractérisée en ce qu'**elle prévoit au moins un premier oeillet (31) dont l'axe longitudinal est placé sur ledit plan sagittal (28) et au moins un second oeillet (32) dont l'axe longitudinal est placé sur ledit plan frontal (27).

7. Prothèse acétabulaire selon la revendication 5, **caractérisée en ce que** chaque oeillet (31, 32) est défini par une pluralité de trous traversants (34) réalisés côte à côte avec un écartement inférieur à leur diamètre, de manière à communiquer entre eux.

8. Prothèse acétabulaire selon la revendication 1, **caractérisée en ce que** lesdits moyens de contrainte peuvent permettre audit élément intermédiaire (12) de pivoter à l'intérieur dudit siège (19) de ladite coque externe (11) sur au moins un plan frontal (27) et sur au moins un plan sagittal (28) sensiblement perpendiculaires entre eux.

9. Prothèse acétabulaire selon la revendication 8, **caractérisée en ce que** lesdits moyens de contrainte peuvent permettre audit élément intermédiaire (12) de pivoter à l'intérieur dudit siège (19) de ladite coque externe (11) par rapport audit plan frontal (27) et/ou audit plan sagittal (28) pour couvrir un angle d'abduction (a) compris entre 0° et 40° et un angle d'antéversion compris entre 0° et 40°.

10. Prothèse acétabulaire selon la revendication 1, **caractérisée en ce que** lesdits moyens de contrainte peuvent permettre audit élément intermédiaire (12) de pivoter et d'être serré à la fois avant et après la fixation de ladite coque externe (11) audit siège acétabulaire de la hanche.

11. Prothèse acétabulaire selon la revendication 1, **caractérisée en ce que** ladite coque externe (11) est pourvue d'au moins une fenêtre (29) et ledit élément intermédiaire (12) est pourvu de trous supplémentaires (30) dans lesquels peuvent être introduites des vis capables d'être vissées au tissu osseux dudit siège acétabulaire de la hanche en passant à travers ladite fenêtre (29), même après que ledit élément intermédiaire (12) ait été définitivement positionné et serré.

12. Prothèse acétabulaire selon la revendication 1, **caractérisée en ce que** ladite coque externe (11) est pourvue d'au moins une griffe (17) et/ou d'ailettes (14) pour ancrer ladite coque externe (11) au siège acétabulaire de la hanche.
